# EUROPÄISCHE PATENTSCHRIFT

(11) **EP 1 510 619 B1**
(45) Veröffentlichungstag und Bekanntmachung des Hinweises auf die Patenterteilung: **14.12.2011**
(21) Anmeldenummer: 04019545.5
(22) Anmeldetag: 18.08.2004
(51) Int. Cl.: D06M 13/292

(54) **Verwendung von Liposomen zur Ausrüstung von Fasern und Textilien**
Use of liposomes for the finishing of fibres and textiles
Utilisation de liposomes pour l'ennoblissement de fibres et produits textiles

(30) Priorität: 27.08.2003 DE 10339358
(43) Veröffentlichungstag der Anmeldung: 02.03.2005
(73) Patentinhaber: Cognis IP Management GmbH, 40589 Düsseldorf (DE)
(72) Erfinder: Wachter, Rolf, 40595 Düsseldorf (DE); Weuthen, Manfred, 40764 Langefeld (DE); Panzer, Claudia, 41515 Grevenbroich (DE); Paff, Erik, 41061 Mönchengladbach (DE)

(56) Entgegenhaltungen:
- EP-A- 0 787 852
- EP-A1- 1 258 240
- DATABASE WPI Section Ch, Week 200306 Derwent Publications Ltd., London, GB; Class A23, AN 2003-066116 XP002353139 & RU 2 188 264 C2 (UNIV IVAN CHEM TECHN) 27. August 2002 (2002-08-27)
- DATABASE WPI Section Ch, Week 200279 Derwent Publications Ltd., London, GB; Class F06, AN 2002-730416 XP002353140 & RU 2 188 263 C2 (UNIV IVAN CHEM TECHN) 27. August 2002 (2002-08-27)

## Beschreibung

Die vorliegende Anmeldung betrifft die Verwendung von Liposomen zur oberflächlichen Ausrüstung von Fasern und/oder textilen Flächengeweben.

Die oberflächliche Ausrüstung von Fasern oder textilen Flächengeweben mit unterschiedlichen Chemikalien ist ein in der Textiltechnik übliches und weit angewendetes Verfahren, um beispielsweise bestimmte Oberflächeneigenschaften von Textilien zu verbessern, beispielsweise deren Hydrophilie oder aber die Textilien und Fasern mit bestimmten Eigenschaften zu versehen, z. B. mit einer Knitterfreiausrüstung oder einem UV-Schutz.

Es besteht allerdings weiterhin der Bedarf, Textilien und Fasern intelligenter auszurüsten, d.h. ihnen Eigenschaften zu verleihen, die bislang aufgrund der im Stand der Technik bekannten Lösungen nicht zugänglich waren.

Die Aufgabe der vorliegenden Erfindung bestand darin, Textilien und Fasern so auszurüsten, dass hautfreundliche oder hautpflegende Substanzen über die Textilien oder Fasern auf die Haut übertragen werden können.

Es hat sich gezeigt, dass der Einsatz von Liposomen die gestellte Aufgabe lösen kann.

Gegenstand der vorliegenden Erfindung ist daher die Verwendung von Liposomen, die gehärtetes Sojaöl-Lecithin enthalten zur oberflächlichen Ausrüstung von Fasern und/oder textilen Flächengeweben.
Aus der EP 787 852 A2 sind Mittel zur Textilbehandlung bekannt, die so genannte Prä-Liposomen enthalten, die auch Phospholipide enthalten können. Die Schrift offenbart die Verwendung dieser Prä-Liposomen zur Herstellung von Färbebädern für Textilien. Die EP 1 258 240 A1 beschreibt eine Vorrichtung zur Abgabe von Pflanzenextrakten auf die menschliche Haut, wobei die Pflanzenextrakte in Form eines Komplexes, der mit Phospholipiden verknüpft ist, Verwendung finden.

Liposomen sind in der Regel kugelförmige Gebilde (Durchmesser typischerweise von 25 nm bis 1 mm) aus einer oder mehreren konzentrischen Lipid-Doppelschichten mit wässerigem Innenraum (Lipidvesikel). Derartige vesikuläre Strukturen lassen sich z.B. durch mechanische Feinstverteilung von Phospholipiden (z. B. Lecithin) in wässerigen Medien herstellen. Phospholipide sind Phosphorsäuredi-, seltener -monoester, die wegen ihrer fettähnlichen Löslichkeitseigenschaften aufgrund der lipophilen und hydrophilen Komponenten zu den Lipiden gerechnet werden und im Organismus als Membranlipide am Aufbau von Schichten-Strukturen, den Membranen, beteiligt sind. Suspendiert man sie, so vereinigen sie sich zu geordneten Aggregaten wie Micellen, Lamellen, Liposomen u. a. Membran-Strukturen. Die Fettsäure-Ketten sind dabei parallel ausgerichtet wie in flüssigen Kristallen, und die Phosphorsäureester-Gruppen weisen in die wässerige Phase.

Bei den erfindungsgemäß vorzugsweise eingesetzten Liposomen, die unter der Handelsbezeichnung Lipocutin® vermarktet werden, handelt es sich um wässerige Dispersionen stabilisierter, unbeladener oder mit Wirkstoff beladener Liposomen auf der Basis von Sojaöl-Lecithin und Cholesterin.

Dabei ist es wesentlich, dass die Liposomen im Sinne der vorliegenden technischen Lehre eine Phospholipidschicht auf Basis von gehärtetem Sojaöl-Lecithin, enthalten.

Bei Lecithinen handelt es sich um Glycero-phospholipide, die aus Fettsäuren, Glycerin, Phosphorsäure und Cholin durch Veresterung gebildet werden. Die in der Natur vorkommenden Lecithine sind wie die eng verwandten Kefaline Derivate der 1,2-Diacylglycerin-3-Phosphorsäuren. Die Art der Phosphorsäure ist entscheidend für die späteren Eigenschaften der Lecithine. Normalerweise ist die gesättigte Fettsäure mit der primären, die ungesättigte mit der sekundären Hydroxylgruppe des Glycerins verestert. Wesentlich für die erfindungsgemäß eingesetzten Lecithine ist der Umstand, dass sie auf hydrierten Fettsäuretriglyceriden basieren, wohingegen die üblicherweise in der Natur vorkommenden Lecithine hohe Anteile ungesättigter Fettsäuren enthalten. Die Liposome der vorliegenden Erfindung sind daher vorzugsweise frei von ungesättigten Lecithinen.

Sojaöl selbst ist ein gelbliches bis braungelbes, fettes, halbtrocknendes Öl, das durch Pressen u./od. Extraktion mit Kohlenwasserstoffen (z. B. Hexan) aus Sojabohnen (Glycine max) oder Sojaschrot gewonnen wird. Ölgehalt der Sojabohnen: 17-22%. Die Zusammensetzung ist in der folgenden Tabelle 1 dargestellt: Es gibt das Fettsäure-Spektrum von Sojaöl wieder; die Angabe der Schwankungsbreite und des häufigsten Durchschnittswertes in Gew.-% der Gesamtfettsäuren in Klammern.

**Tabelle 1**

| | | |
|---|---|---|
| C < 14 | <0,1 | |
| C 14 : 0 | <0,5 | |
| C 16 : 0 | 7,0-14 | (10) |
| C 16 : 1 | <0,5 | |
| C 18 : 0 | 1,4-4,5 | (4,0) |
| C 18 : 1 | 19-30 | (21) |
| C 18 : 2 | 44-62 | (56) |
| C 18 : 3 | 4-11 | (8) |
| C 20 : 0 | <1,0 | (0,5) |
| C 20 : 1 | <1,0 | (0,5) |
| C 22 : 0 | <0,5 | |

55-65% der Gesamtfettsäuren des Sojaöl sind mehrfach ungesättigte Fettsäuren. Der Sterin-Gehalt von Sojaöl beträgt durchschnittlich 0,37% (davon Cholesterin 0,3-0,5%). Neben Cholesterin finden sich in Sojaöl. vor allem Ergost-5-en-3b-ol, Campesterin und Sitosterin. Durch Raffination läßt sich der Sterin-Gehalt um ca. 30% senken. Darüber hinaus enthält Sojaöl freie Fettsäuren, Lecithin u. bis zu 0,8% Tocopherol. Sojäöl hat eine Dichte von 0,916-0,922, einen Schmelzpunkt von -15 bis -8°C, einen Festpunkt von 282 °C, die Verseifungszahl beträgt 188-195, die Iodzahl 120-136 und die Säurezahl liegt im Bereich von 0,3-3,0, der unverseifbarer Anteil beträgt typischerweise 0,5-1,5%.

Die Liposomen der vorliegenden Erfindung enthalten weiterhin vorzugsweise Cholesterine. Cholesterin, systematisch 5-Cholesten-3b-ol, oder Cholesterol, Summenformel: C₂₇H₄₆O, Molgewicht 386,66; bildet rein farblose Blättchen, Dichte 1,052, Schmelzpunkt 148,5 °C, Siedpunkt 360 °C (geringe Zersetzung). Es ist ein in Wasser praktisch nicht, in Alkohol in der Kälte wenig, in der Wärme besser, in Ether, Benzol, Petrolether lösliche Verbindung. In den Liposomen der vorliegenden Erfindung kommt es vorzugsweise in Mengen von etwa 50 Gew.-% vor. Es ist weiterhin bevorzugt, dass die Liposomen der vorliegenden Erfindung zusätzlich noch Alkyphosphate enthalten, vorzugsweise Dialkylphosphate und insbesondere Dicetylphosphat.

Die Liposomen der vorliegenden Erfindung weisen vorzugsweise einen Durchmesser von 25 nm bis 1 mm auf und insbesondere liegt die mittlere Teilchengröße im Bereich von 100 bis 800 nm und vorzugsweise von 100 nm bis 500 nm und insbesondere von 150 bis 300 nm. Ein wesentlicher vorteilhafter Aspekt der vorliegenden technischen Lehre besteht darin, dass die Liposomen gemäß der obigen Beschreibung vorzugsweise in ihrem Inneren hautpflegende Inhaltsstoffe enthalten. Üblicherweise umschließen die Liposomen der vorliegenden Erfindung einen wässrigen Kern, der geeignet ist, eine Vielzahl von dem Fachmann bekannten Inhaltsstoffen aufzunehmen. Es kann sich hierbei um hautpflegende Substanzen aller Art handeln, sofern sie geeignet sind, in einer wässrigen Umgebung stabil von Liposomen eingeschlossen zu werden. Bespiele für derartige Inhaltsstoffe sind Proteinhydrolysate, Moisturizer, Polymere und besonders geeignet, Parfümöle. Es gilt, das insbesondere hydrophile, wasserdispergierbare Substanzen geeignet sind, in den Liposomen eingeschlossen zu werden. Es ist auch möglich lipophile Substanzen in die Liposome zu integrieren, wobei diese allerdings in der Membrane und nicht im wassergefüllten Hohlraum enthalten sind. Die Liposomen der vorliegenden Erfindung müssen allerdings frei von anionischen Verbindungen sein.

Die Liposomen im Sinne der vorliegenden Erfindung können vorzugsweise in Form wässriger Mittel eingesetzt werden, enthaltend die Liposomen sowie vorzugsweise mindestens einen Lösungsvermittler, der vorzugsweise ausgewählt ist aus der Gruppe Monoalkohole mit 2 bis 6 C-Atomen. Ein besonders geeigneter Alkohol im Sinne der vorliegenden Erfindung stellt Ethanol dar. Üblicherweise enthalten solche Mittel 95 bis 50 Gew.-% Wasser sowie 1 bis 50 Gew.-% des Alkohols.
Die Liposomen sind in Anteilen von 0,1 bis 15 Gew.-%, vorzugsweise von 1 bis 5 Gew.-%, insbesondere von 1 bis 2,5 Gew.-% enthalten. Ein typisches Mittel im Sinne der vorliegenden Erfindung enthält dann 60 bis 80 Gew.-% Wasser, 10 bis 25 Gew.-% eines niederen organischen Alkohols, vorzugsweise Ethanol und 5 bis 15 Gew.-% an Liposomen. Es sind aber auch rein wässerige Mittel möglich. Die Liposomen liegen in der Regel feindispergiert in der kontinuierlichen wässerigen Phase vor.

Weiterhin bevorzugt ist die Verwendung von demineralisiertem Wasser zur Herstellung der Mittel. Zur Konservierung können übliche Konservierungsmittel verwendet werden, wobei Phenoxyethanol als bevorzugtes Mittel genannt wird. Die Mittel können neben den Liposomen noch andere Inhaltsstoffe aufweisen, vorzugsweise Tenside und weichmachende Substanzen, beispielsweise quaternierte Ammoniumverbindungen. Es kann vorteilhaft sein, wenn die Liposomen im Sinne der vorliegenden Erfindung mit Tensiden zusammen formuliert werden sollen, auf die Mitverwendung von anionischen Tensiden zu verzichten. Bevorzugt ist aber die Mitverwendung kationischer und/oder nichtionischer Tenside.

Die wässrigen Mittel im Sinne der vorliegenden Erfindung weisen pH-Werte im Bereich von 4,5 bis 7,0, insbesondere 5 bis 7 und vorzugsweise im Bereich von 5,5 bis 6,5 auf.

Des weiteren wird ein Verfahren beansprucht zur Auftragung hautfreundlicher Substanzen auf die Haut, wobei man die Haut in Kontakt bringt mit liposomhaltigen Fasern oder Textilien gemäß der obigen Beschreibung und die Liposomen von den Fasern oder Textilien durch mechanische und/oder thermische Belastung geöffnet werden und somit die hautaktiven Substanzen wie auch die Phospholipide auf die Haut übertragen werden können. Die Phospholipide aus den Liposomen können die Penetration von hautpflegenden Stoffen erhöhen und somit einen Zusatznutzen verleihen.
In der Praxis bedeutet dies, dass Textilien oder Fasern im Sinne der vorliegenden Erfindung bei Erwärmung auf Hauttemperatur oder darüber, d. h. > 35° C, einen Trocknungsprozess durchlaufen, bei dem die liposomale Struktur aufgebrochen und die Inhaltsstoffe feinstverteilt auf die Oberfläche der Fasern gelangen können. Ohne an eine bestimmte Theorie gebunden zu sein geht die Anmelderin davon aus, dass sich so die Liposome öffnen und sich auf den Fasern ein dünner Film der Inhaltsstoffe der Liposome und der Phospholipide ausbildet. Dieser Film auf den Fasern gerät in Kontakt mit der Haut und wird dann durch mechanische Prozesse übertragen.

Die erfindungsgemäße Ausrüstung von Fasern und/oder textilen Flächengeweben eröffnet eine Vielzahl von Applikationsmöglichkeiten. Die Ausrüstung der Fasern oder Textilien selber kann auf aller dem Fachmann Art und Weise erfolgen, also beispielsweise durch eine Spray- oder durch Tauchbehandlung. Die Liposomen können aber auch in maschinellen Waschverfahren eingesetzt werden, beispielweise durch Einspülen in einer Haushaltswaschmaschine.

Textilien, die im Sinne der vorliegenden Erfindung mit Liposomen oberflächlich ausgerüstet sind, vermögen ihren Trägem eine Vielzahl positiver Eigenschaften zu vermitteln, insbesondere Pflegeeffekte auf der Haut, wobei ein wesentlicher positiver Aspekt ist, dass durch die Feinstverteilung der Liposomen und deren Inhaltsstoffen kein fettiger oder schmieriger Eindruck beim Anwender entsteht. Aufgrund der geringen Konzentration der aktiven Inhaltsstoffe dringen diese kurzfristig in die Haut ein und können so ihre pflegenden Wirkungen schnell und wirkungsvoll erzielen. Durch den kontinuierlichen Transport von hautpflegenden Substanzen aus den Liposomen auf die Haut kommt es zu einer langanhaltende Wirkung im Vergleich zu einer direkten Applikation der Hautpflegesubstanzen. Ein zusätzlicher Aspekt besteht darin, dass aufgrund der natürlich vorhandenen Feuchtigkeit auf der Oberfläche der Haut durch entsprechend abgestimmte Inhaltsstoffe der Liposomen, beispielsweise durch den Zusatz von Emulgatoren zu den pflegenden Inhaltsstoffen auf der Hautoberfläche beim Träger der Textilien Emulsionen gebildet werden, was eine Vielzahl von Formulierungen für pflegende Inhaltsstoffe ermöglicht.

Die Liposomen gemäß der obigen Beschreibung eignen sich vorzugsweise zur Ausrüstung von Fasern und/oder Textilien die ganz oder teilweise Wolle, Baumwolle, Seide, Cellulose und/oder synthetische Fasern enthalten, aber auch für die Ausrüstung von Papier. Bevorzugt ist insbesondere die Ausrüstung von Baumwolle, Baumwollemischgeweben aber auch synthetischen Fasern, vorzugsweise Nylonfasern.

### Beispiele

Es wurde eine wässerige Liposomendispersion (Plantatex® Lip, Fa. Cognis) verwendet, welche 2 Gew.-% Lipide enthielten. Die Liposomen bestanden aus hydriertem Sojalecithin, Cholesterin und Dicetylphosphat. Die mittlere Teilchengröße lag bei 400 nm. Die wässerige Liposomendispersion wies einen pH-Wert von 6,5 auf.

### Applikationsversuche

### 1. Pump Spray:

Es wurde ein wässeriges Mittel hergestellt, enthaltend 10 Gew.-% der wässerigen Liposomendispersion und 0,4 Gew.-% Phenoxyethanol, der Rest auf 100 Gew.-% war Wasser. Dieses Mittel hatte eine Viskosität von < 50 mPas und einen pH-Wert von 6,6. Das Mittel war bei - 5, 8, 23 und 40 °C mindestens 12 Wochen lagerstabil. Das Mittel wurde als Pumpspray 20 mal auf ein 20 x 30 cm großes Testgewebe (Baumwolle Wfk 10A oder Wolle Wfk60A oder Baumwollwirkware Wfk80A) appliziert. Weiterhin wurden Versuche an Seide 70A und Lenzing Viskose durchgeführt. Das Testgewebe wurde bei Raumtemperatur getrocknet. Tabelle 1 gibt den Gehalt an Liposomen auf den Testgeweben wieder:

**Tabelle 1:**

| | **Gewicht vor Applikation** | **Gewicht nach Applikation** | **Menge Formulierung** | **Gehalt an Liposomen mg/cm²** |
|---|---|---|---|---|
| Baumwolle Wfk 10A | 7,38 | 9,50 | 2,12 | 0,007 |
| Wolle Wfk 60A | 9,31 | 12,29 | 2,98 | 0,010 |
| Wirkware Wfk 80A | 10,64 | 13,72 | 3,08 | 0,010 |

| | | | | |
|---|---|---|---|---|
| Die Liposomen ließen sich anschließend durch Wasser wieder von den Testgeweben eluieren, wobei die Liposomen im wässerigen Eluat nachweisbar waren. | | | | |

Diese Mittel wurden auf Textilien aus Baumwolle Wfk10A aufgesprüht. Anschließend wurden die sensorischen Eigenschaften (z.B. Glanz, Weichheit, Elastizität, Tragekomfort, Trocknungsverhalten, Härte) der Textilien durch eine Gruppe von 10 Testpersonen gegen das unbehandelte (d.h. nur mit Wasser behandelte) Textil geprüft. Die erfindungsgemäß behandelten Textilien wiesen bei der Elastizität, Härte und Weichheit bessere Eigenschaften auf, als die nur mit Wasser behandelten Textilien.

### 2. CareBalm

Als CareBalm werden Rezepturen bezeichnet, die über die Einspülkammer einer Waschmaschine appliziert werden können, um die Inhaltsstoffe während des Waschganges auf die Textilien aufzubringen. Es wurden zwei Formulierungen untersucht, die neben der wässerigen Liposomendispersion noch handelsübliche Weichmacher auf Basis handelsüblicher quaternierter Ammoniumverbindungen (Dehyquart AU, Fa. Cognis) enthielten. Die Rezeptur ist in Tabelle 2 aufgeführt:

**Tabelle 2:**

| | **Formulierung A Gew.-%** |
|---|---|
| N-Methyl-N,N-bis(acyloxyethyl)-N-(2-hydroxyethyl)ammonium-methosulfat | 2 |
| Phenoxyethanol | 0,4 |
| Liposomendispersion | 10 |
| Wasser | Rest |

Die Formulierungen wurden hergestellt, indem man die quaternierten Ammoniumverbindungen (QAV) in warmes Wasser einrührte, und anschließend bei 30 °C die Liposomendispersion zumischte. Der pH-Wert dieser Formulierungen liegt bei 4,3, die Viskosität war < 50 mPas.
Das Mittel aus Tabelle 1 wurden in einer handelsüblichen Waschmaschine (Miele W467) im Softenerschritt des Waschprogramm appliziert. Dabei wurden pro Einspülzyklus 80 g der obigen Formulierungen auf die Testgewebe aus Baumwolle WfK 10A bzw. Baumwollwirkware 80A appliziert. Auf den Textilien konnte anschließend die Liposomen nachgewiesen werden.

Für die sensorische Prüfung wurde ein Testgewebe aus Frottee wie oben beschrieben mit den Liposomen ausgerüstet. Diese Textil wurde durch ein Panel von 11 Testpersonen verglichen mit einem Frottee-Textil welches mit einer Rezeptur gemäß Formulierung A, aber ohne die Liposomenkomponente ausgerüstet worden war (Referenz). Der Gehalt an QAV war dabei 4 Gew.-%. Im direkten Vergleich wurde das erfindungsgemäß ausgerüstete Textil im Trocknungsverhalten deutlich besser benotet als das Vergleichstextil.

In einem weiteren Test wurde eine 10 Gew.-%ige wässerige Liposomendispersion mittels Tauchapplikation auf Nylonstrümpfe aufgebracht und anschließend an der Luft getrocknet. Es konnten durch Rückwägung 0,14 Gew.-% an Liposomen (bezogen auf eingesetzte Aktivsubstanz) auf den Strümpfen nachgewiesen werden. Bei einer Beurteilung durch ein Panel von 11 Personen wurden die erfindungsgemäß ausgerüsteten Strümpfe als Weicher und von Tragekomfort her als angenehmer eingestuft als die nur mit Wasser behandelten Vergleichsstrümpfe.

## Patentansprüche

1. Verwendung von Liposomen zur oberflächlichen Ausrüstung von Fasern und/oder textilen Flächengeweben, **dadurch gekennzeichnet, dass** die verwendeten Liposomen gehärtetes Sojaöl-Lecithin enthalten.

2. Verwendung nach Anspruch 1, **dadurch gekennzeichnet, dass** die verwendeten Liposomen zusätzlich noch Cholesterin enthalten.

3. Verwendung nach den Ansprüchen 1 bis 2, **dadurch gekennzeichnet, dass** die verwendeten Liposomen zusätzlich noch Alkyphosphate enthalten, vorzugsweise Dialkylphosphate und insbesondere Dicetylphosphat.

4. Verwendung nach den Ansprüchen 1 bis 3, **dadurch gekennzeichnet, dass** die Liposomen einen mittleren Durchmesser von 25 nm bis 1 mm, vorzugsweise von 100 nm bis 500 nm aufweisen.

5. Verwendung von Liposomen gemäß den Ansprüchen 1 bis 4, **dadurch gekennzeichnet, dass** die Liposomen hautpflegende Inhaltsstoffe in ihrem Inneren umschlossen enthalten.

6. Verwendung von Liposomen gemäß den Ansprüchen 1 bis 5 zur Ausrüstung von Fasern und/oder Textilien die ganz oder teilweise Wolle, Baumwolle, Cellulose und/oder synthetische Fasern enthalten.

7. Fasern oder Textilien, die auf ihren Oberflächen Liposomen aufweisen, die gehärtetes Sojaöl-Lecithin sowie optional Cholesterin und/oder Alkylphosphate enthalten.

8. Verfahren zur Auftragung von hautfreundlichen Substanzen auf die Haut, **dadurch gekennzeichnet, dass** man die Haut in Kontakt bringt mit liposomhaltigen Fasern oder Textilien und dabei die hautfreundlichen Inhaltsstoffe der Liposomen von den Fasern oder Textilien durch mechanische Belastung der Liposomen auf die Haut übergehen, **dadurch gekennzeichnet, dass** die verwendeten Liposomen gehärtetes Sojaöl-Lecithin enthalten.

9. Mittel, enthaltend Wasser, einen Lösungsvermittler, vorzugsweise einen organischen Monoalkohol mit 2 bis 6 C-Atomen, sowie Liposomen, die gehärtetes Sojaöl-Lecithin und optional Cholesterin und/oder Alkylphosphate enthalten.

## Claims

1. Use of liposomes for the surface finishing of fibers and/or flat textiles, **characterized in that** the liposomes used contain hydrogenated soybean oil lecithin.

2. Use according to Claim 1, **characterized in that** the liposomes used additionally contain cholesterol.

3. Use according to Claims 1 to 2, **characterized in that** the liposomes used additionally contain alkyl phosphates, preferably dialkyl phosphates and, in particular, dicetyl phosphate.

4. Use according to Claims 1 to 3, **characterized in that** the liposomes have a mean diameter of 25 nm to 1 mm, preferably 100 nm to 500 nm.

5. Use of liposomes according to Claims 1 to 4, **characterized in that** the liposomes contain skin-care ingredients encapsulated in their interior.

6. Use of liposomes according to Claims 1 to 5 for finishing fibers and/or textiles completely or partly containing wool, cotton, cellulose and/or synthetic fibers.

7. Fibers or textiles with liposomes on their surfaces which contain hydrogenated soybean oil lecithin and optionally cholesterol and/or alkyl phosphates.

8. Process for applying skin-friendly substances to the skin, **characterized in that** the skin is contacted with liposome-containing fibers or textiles and, in the process, the skin-friendly ingredients of the liposomes pass from the fibers or textiles onto the skin by mechanical pressure on the liposomes, **characterized in that** the liposomes used contain hydrogenated soybean oil lecithin.

9. Preparations containing water, a solubilizer, preferably an organic C₂₋₆ monoalcohol, and liposomes which contain hydrogenated soybean oil lecithin and optionally cholesterol and/or alkyl phosphates.

## Revendications

1. Utilisation de liposomes pour l'apprêt superficiel de fibres et/ou de tissus plans textiles, **caractérisée en ce que** les liposomes utilisés présentent de la lécithine d'huile de soja durcie.

2. Utilisation selon la revendication 1, **caractérisée en ce que** les liposomes utilisés contiennent en outre encore du cholestérol.

3. Utilisation selon les revendications 1 à 2, **caractérisée en ce que** les liposomes utilisés contiennent en outre encore des alkylphosphates, de préférence des dialkylphosphates et en particulier du dicétylphosphate.

4. Utilisation selon les revendications 1 à 3, **caractérisée en ce que** les liposomes présentent un diamètre moyen de 25 nm à 1 mm, de préférence de 100 nm à 500 nm.

5. Utilisation de liposomes selon les revendications 1 à 4, **caractérisée en ce que** les liposomes contiennent, en leur intérieur, de manière enrobée, des constituants de soin de la peau.

6. Utilisation de liposomes selon les revendications 1 à 5 pour l'apprêt de fibres et/ou de textiles qui contiennent, totalement ou en partie, de la laine, du coton, de la cellulose et/ou des fibres synthétiques.

7. Fibres ou textiles qui présentent, en leurs surfaces, des liposomes qui contiennent de la lécithine d'huile de soja durcie ainsi qu'éventuellement du cholestérol et/ou des alkylphosphates.

8. Procédé pour l'application, sur la peau, de substances douces pour la peau, **caractérisé en ce qu'**on met la peau en contact avec des fibres ou des textiles contenant des liposomes et les constituants doux pour la peau des liposomes sont transférés des fibres ou des textiles à la peau par sollicitation mécanique des liposomes, **caractérisé en ce que** les liposomes utilisés présentent de la lécithine d'huile de soja durcie.

9. Agent, contenant de l'eau, un promoteur de solubilisation, de préférence un monoalcool organique comprenant 2 à 6 atomes de carbone, ainsi que des liposomes, qui contiennent de la lécithine d'huile de soja durcie et éventuellement du cholestérol et/ou des alkylphosphates.
